# EUROPEAN PATENT SPECIFICATION

(11) **EP 2 713 162 B1**
(45) Date of publication and mention of the grant of the patent: **19.10.2016**
(21) Application number: 13186447.2
(22) Date of filing: 27.09.2013
(51) Int. Cl.: G01N 33/533, G01N 33/543, G01N 33/58, G01N 33/552, G01N 33/12

(54) **Measurement method and measurement kit of antibiotics concentration**
Messverfahren und Messkit für die Konzentration von Antibiotika
Procédé de mesure et kit de mesure de concentration d'antibiotiques

(30) Priority: 27.09.2012 KR 20120108074
(43) Date of publication of application: 02.04.2014
(73) Proprietor: Industry-Academic Cooperation Foundation Dankook University, Yongin-si, Gyeonggi-do 448-701 (KR); Republic of Korea (Ministy of Food and Drug Safety), Chungcheongbuk-do 363-700 (KR); Specialty Lab Solution Co., Ltd., Gyeonggi-do (KR)
(72) Inventor: Lim, Heung Bin, 463-781 Gyeonggi-do (KR); Ko, Jung Ah, 143-893 Seoul (KR); Kim, Su Ji, 423-738 Gyeonggi-do (KR); Park, Ki Hwan, 110-848 Seoul (KR); Kim, Kyu Heon, 336-750 Chungcheongnam-do (KR); Lee, Hwa Jung, 158-757 Seoul (KR); Han, Sang Bae, 122-751 Seoul (KR)
(74) Representative: Zimmermann, Tankred Klaus

(56) References cited:
- JP-A- H07 151 756
- YOSHIO KOBAYASHI ET AL: "Silica-coating of fluorescent polystyrene microspheres by a seeded polymerization technique and their photo-bleaching property", COLLOIDS AND SURFACES A: PHYSICOCHEMICAL AND ENGINEERING ASPECTS, vol. 242, no. 1-3, 15 June 2004 (2004-06-15), pages 47-52, XP055098044, ISSN: 0927-7757, DOI: 10.1016/j.colsurfa.2004.04.052
- WACHIRA TANSUB ET AL: "Synthesis of Antibodies-Conjugated Fluorescent Dye-Doped Silica Nanoparticles for a Rapid Single Step Detection of Campylobacter jejuni in Live Poultry", JOURNAL OF NANOMATERIALS, vol. 22, no. 1, 1 January 2012 (2012-01-01), pages 1743-7, XP055098065, ISSN: 1687-4110, DOI: 10.1016/j.talanta.2007.12.048
- HÉCTOR FONT ET AL: "Immunochemical Assays for Direct Sulfonamide Antibiotic Detection In Milk and Hair Samples Using Antibody Derivatized Magnetic Nanoparticles", JOURNAL OF AGRICULTURAL AND FOOD CHEMISTRY, vol. 56, no. 3, 13 February 2008 (2008-02-13), pages 736-743, XP055097562, ISSN: 0021-8561, DOI: 10.1021/jf072550n

## Description

### CLAIM FOR PRIORITY

This application claims priority to Korean Patent Application No. 2012-0108074 filed on September 27, 2012 in the Korean Intellectual Property Office (KIPO).

### BACKGROUND

### 1. Technical Field

Example embodiments of the present invention relate in general to a method and kit for measuring a concentration of an antibiotic.

### 2. Related Art

Antibiotics are metabolites which are produced by microorganisms, and thus can be used in small amounts to inhibit the growth of other microorganisms or kill other microorganisms. Therefore, antibiotics have been widely used in domestic meat animals such as pigs, chickens, and cattle. However, since the antibiotics used in the domestic meat animals are not easily decomposed in an animal body, the antibiotics can be easily introduced into the animal body when the domestic meat animals are ingested. The antibiotics introduced into the animal body may influence the central nervous system, and elicit resistance of pathogens to medical supplies as well.

Therefore, monitoring a concentration of the antibiotics in the domestic meat animals is considered to be one of the key items in food safety testing.

JP H 07 151756 A describes a method for measuring an antibody. To quickly and easily separate unreacted matters form a reaction product and clean the unreacted matters magnetic particles and fluorescent particles are used. A first reagent containing insoluble magnetic particles carrying the antibody meeting an antibody to be measured and the antibody to be measured are caused to react to each other in a liquid medium contained in a reaction vessel. After reaction, the magnetic particles are attracted to the wall of the vessel by utilizing the action of a magnetic field and the liquid medium is removed from the vessel. Then a second reagent containing insoluble fluorescent pigment-labeled particles carrying an antigen which is specifically bonded to the antibody to be measured and the magnetic particles attracted to the wall of the vessel are caused to react to each other in the liquid medium. Thereafter, the magnetic particles are again attracted to the wall of the vessel by utilizing the action of the magnetic field and the liquid medium and unreacted pigment-labeled particles are removed, and then, the fluorescent intensity of the pigment-labeled particles which have reacted to the magnetic particles is measured.

YOSHIO KOBAYASHI ET AT: "Silica-coating of fluorescent polystyrene microspheres by a seeded polymerization technique and their photo-bleaching property" COLLOIDS AND SURFACES A: PHYSIOCHEMICAL AND ENGINEERING ASPECTS, vol. 242, no. 1-3. 15 June 2004, pages 47-53, describe silica-coating of polystyrene microspheres incorporated with fluorescence dyes (fluorescent microspheres) by means of a seeded polymerization technique based on the Stöber method. The silica-coating of the fluorescent microspheres is performed in the presence of 0-10 g/l polyvinylpyrrolidone (PVP), 1.13-17 M water, 0-1.2 M aqueous ammonia and 0.00038-0.2 M tetraethoxyorthosilicate (TEOS). The addition of PVP suppresses the generation of free silica particles and improves the uniformity of shell thickness. The silica shell thickness increases from 13 to 138 nm with an increase in TEOS concentration at 10 g/l PVP, 0.4 M aqueous ammonia and 10.9 M water. The thickness also increases with the ammonia concentration and the water concentration. The silica-coated fluorescence microspheres show more stable fluorescence to laser-irradiation than uncoated microspheres.

WACHIRA TANSUB ET AL: "Synthesis of Antibodies-Conjugated Fluorescent Dye-Doped Silica Nanoparticles for a Rapid Single Step Detection of Campylobacter jejuni in Live Poultry", JOURNAL OF NANOMATERIALS, vol. 22, no. 1, 1. January 2012, pages 1743-7 describe the preparation of antibodies-conjugated fluorescent dye-doped silica nanoparticles (FDS-NPs) to detect Campylobacter jejuni cells under a fluorescence microscope. The particles prepared by sol-gel microemulsion techniques have a round shape with an average size of 43 ± 4 nm. They are highly photo stable and emit strong orange fluorescent for 60 min. Both amine- and carboxyl-functionalized properties are evident from FTIR and FT Raman spectra. The FDS-NPs conjugated with antibodies against C. jejuni are well dispersed in PBS solution at 20 mM of NaCl. The conjugation with monoclonal antibodies against C. jejuni was successful. The direct observation of the antibodies-conjugated FDS-NPs- that bounds C. jejuni with Petroff Hausser counting chamber at 40x is clear. The different focus lengths clearly separate bound and unbound FDS-NPs under the microscope.

HECTOR FONT ET AL: "Immunochemical Assays for Direct Sulfonamide Antibiotic Detection In Milk and Hair Samples Using Antibody Derivatized Magnetic Nanoparticles", Journal of Agricultural and Food Chemistry, vol. 56, no. 3, 13 February 2008, pages 736-743 describes two direct enzyme-linked immunosorbent assays (ELISAs) for the detection of sulfonamide antibiotic residues in milk samples. One of them is using magnetic nanoparticles (MNP) for target capture/enrichment (Ab-MNP-ELISA), and the second is performed using microtiter plates. Selective polyclonal antibodies, raised against 5-[6-(4-amino-benzenesulfonylamino)-pyridin-3-yl]-2-methyl-pentanoic acid (SA1), used in combination with an enzyme tracer prepared with the same hapten, allowed to reach a limit of detection (LOD) lower than 0.5 µg L-1 for both ELISA formats. Sulfapyridine, sulfamethoxypyridazine, sulfathiazole, and sulfachloropyridazine are detected below the maximum residue limits established by the European Union for these antibiotics in milk (100 µg L-1). Matrix effects and accuracy studies performed with full-cream milk and hair extracts indicate a lack of interference from these sample matrices and very good recovery values, especially when using the Ab-MNP format. Milk samples and hair extracts are measured without any previous treatment.

### SUMMARY

Accordingly, example embodiments of the present invention are provided to substantially obviate one or more problems due to limitations and disadvantages of the related art.

Example embodiments of the present invention provide a method of measuring a concentration of an antibiotic, which includes simple processes and has a low detection limit.

Also, example embodiments of the present invention provide a kit for measuring a concentration of an antibiotic, which includes simple processes and has a low detection limit.

In some example embodiments, a method of measuring a concentration of an antibiotic includes preparing magnetic particles bound to an antibiotic, preparing silica-coated fluorescent particles to which at least one antibody of the antibiotic is bound, allowing the magnetic particles to react with the silica-coated fluorescent particles, and irradiating the reacted silica-coated fluorescent particles with laser beams.

In other example embodiments, a kit for measuring a concentration of an antibiotic includes a first measurement unit comprising magnetic particles to which a functional group capable of binding to an antibiotic is bound, and a second measurement unit comprising silica-coated fluorescent particles to which an antibody of the antibiotic is bound.

### BRIEF DESCRIPTION OF DRAWINGS

Example embodiments of the present invention will become more apparent by describing in detail example embodiments of the present invention with reference to the accompanying drawings, in which:
FIG. 1 is a diagram showing chemical formulas of the antibiotics enrofloxacin, ciprofloxacin, salinomycin, and sulfathiazole;
FIG. 2 is a conceptual diagram schematically showing a method of measuring a concentration of enrofloxacin according to one example embodiment of the present invention;
FIG. 3 is a flowchart schematically showing a method of measuring a concentration of an antibiotic according to one example embodiment of the present invention;
FIG. 4 is a graph showing measurement results of fluorescein isothiocyanate (FITC) and silica-coated FITC particles in a pH stability test;
FIG. 5 is a graph showing measurement results of FITC, core particles composed of FITC, and silica-coated FITC particles in a photobleaching stability test;
FIG. 6 is a graph showing measurement results of FITC in a metal quenching stability test; and
FIG. 7 is a graph showing measurement results of silica-coated FITC particles in a metal quenching stability test.

### DESCRIPTION OF EXAMPLE EMBODIMENTS

Example embodiments of the present invention are disclosed herein. However, specific structural and functional details disclosed herein are merely representative for purposes of describing example embodiments of the present invention, however, example embodiments of the present invention may be embodied in many alternate forms and should not be construed as limited to example embodiments of the present invention set forth herein.

Accordingly, while the invention is susceptible to various modifications and alternative forms, specific embodiments thereof are shown by way of example in the drawings and will herein be described in detail. It should be understood, however, that there is no intent to limit the invention to the particular forms disclosed, but on the contrary, the invention is to cover all modifications, equivalents, and alternatives falling within the scope of the invention. Like numbers refer to like elements throughout the description of the figures.

It will be understood that, although the terms first, second, etc. may be used herein to describe various elements, these elements should not be limited by these terms. These terms are only used to distinguish one element from another. For example, a first element could be termed a second element, and, similarly, a second element could be termed a first element, without departing from the scope of the present invention. As used herein, the term "and/or" includes any and all combinations of one or more of the associated listed items.

It will be understood that when an element is referred to as being "connected" or "coupled" to another element, it can be directly connected or coupled to the other element or intervening elements may be present. In contrast, when an element is referred to as being "directly connected" or "directly coupled" to another element, there are no intervening elements present. Other words used to describe the relationship between elements should be interpreted in a like fashion (*i.e.,* "between" versus "directly between," "adjacent" versus "directly adjacent," etc.).

The terminology used herein is for the purpose of describing particular embodiments only and is not intended to be limiting of the invention. As used herein, the singular forms "a," "an" and "the" are intended to include the plural forms as well, unless the context clearly indicates otherwise. It will be further understood that the terms "comprises," "comprising," "includes" and/or "including," when used herein, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

It should also be noted that in some alternative implementations, the functions/acts noted in the blocks may occur out of the order noted in the flowcharts. For example, two blocks shown in succession may in fact be executed substantially concurrently or the blocks may sometimes be executed in the reverse order, depending upon the functionality/acts involved.

Hereinafter, example embodiments of the present invention will be described with reference to the accompanying drawings.

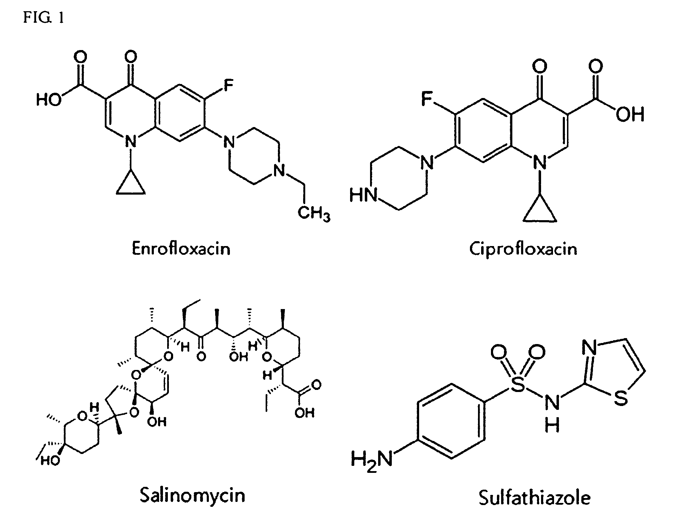

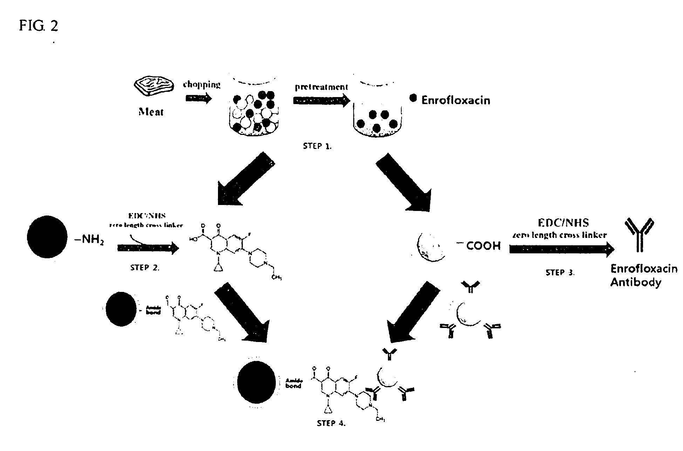

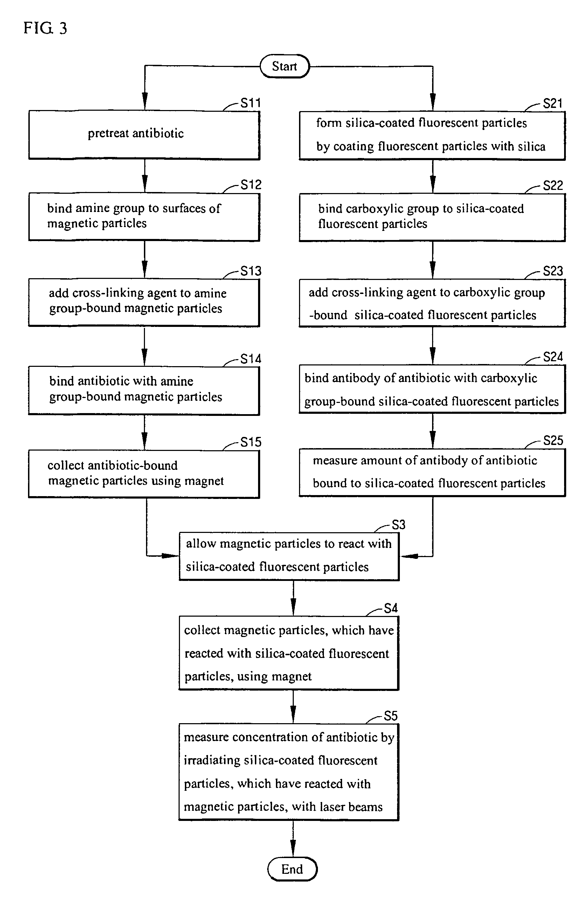

FIG. 1 is a diagram showing chemical formulas of the antibiotics enrofloxacin, ciprofloxacin, salinomycin, and sulfathiazole. FIG. 2 is a conceptual diagram schematically showing a method of measuring a concentration of enrofloxacin according to one example embodiment of the present invention. FIG 3 is a flowchart schematically showing a method of measuring a concentration of an antibiotic according to one example embodiment of the present invention. Hereinafter, the method of measuring a concentration of an antibiotic will be described in further detail with reference to FIGS. 1, 2 and 3.

The method of measuring a concentration of an antibiotic according to one example embodiment of the present invention includes preparing magnetic particles bound to an antibiotic, preparing silica-coated fluorescent particles to which at least one antibody of the antibiotic is bound, allowing the magnetic particles to react with the silica-coated fluorescent particles (S3), and irradiating the silica-coated fluorescent particles, which have reacted with the magnetic particles, with laser beams (S5).

The antibiotic may be selected from the group consisting of enrofloxacin, ciprofloxacin, salinomycin, and sulfathiazole.

Before preparation of the magnetic particles bound to the antibiotic, the method according to one example embodiment may further include pretreating the antibiotic (S11). That is, according to example embodiment of the present invention, large amounts of proteins and fats present in meat of domestic animals such as a pig, a chicken, and cattle may first be removed, and an antibiotic may be extracted from the meat. An exemplary method of removing proteins from the meat may include introducing acetonitrile into the meat, followed by subjecting the meat to vortexing and ultrasonication. Also, an exemplary method of removing fats from the meat may include a method including collecting a supernatant from a solution from which the proteins are removed, adding hexane at the same volume ratio as the acetonitrile, and subjecting the supernatant to vortexing and ultrasonication. To remove the residual impurities, removal of impurities having a size of 0.2 µm or more using a syringe filter may be further performed.

The preparation of the magnetic particles bound to the antibiotic may include binding an amine group to surfaces of the magnetic particles (S12), and forming an amide bond between the amine group-bound magnetic particles and the antibiotic (S14). In this case, the binding of the amine group of the magnetic particles (S12) includes adding 3-aminopropyltriethoxysilane (3-APTES) and tetraethyl orthosilicate (TEOS) to the magnetic particles, and agitating the resulting mixture.

Adding a cross-linking agent (S 13) may be further performed after binding the amine group to the surfaces of the magnetic particles (S12). In this case, at least one selected from the group consisting of N-(3-dimethylaminopropyl)-N'-ethylcarbodiimide (EDC), and N-hydroxysuccinimide (NHS) may be used as the cross-linking agent.

The method of measuring a concentration of an antibiotic according to one example embodiment of the present invention may further include collecting the magnetic particles forming the amide bond with the antibiotic using a magnet (S 15) after formation of the amide bond between the amine group-bound magnetic particles and the antibiotic. When the magnetic particles are collected using the magnet, an increase in concentration of the antibiotic may be facilitated, which makes it favorable to measure a concentration of the antibiotic.

The preparation of the silica-coated fluorescent particles may include forming silica-coated fluorescent particles by coating fluorescent particles including a first fluorescent material with silica (S21), and binding at least one antibody of the antibiotic to the silica-coated fluorescent particles. The first fluorescent material may be at least one material selected from the group consisting of fluorescein isothiocyanate (FITC), and cyanine (Cy).

Here, the fluorescent particles including the first fluorescent material may be prepared by completely dissolving the first fluorescent material in an organic solvent by adding the first fluorescent material in the organic solvent and ultrasonicating the first fluorescent material, and agitating the organic solvent in which the first fluorescent material is dissolved in a light-shielded state.

The forming of the silica-coated fluorescent particles by coating the fluorescent particles including the first fluorescent material with silica (S21) may include forming a reverse microemulsion system by mixing a docusate sodium salt and deionized water (DIW) with heptane and agitating the resulting mixture, adding the fluorescent particles including the first fluorescent material to the reverse microemulsion system while stirring, and adding TEOS and NH₄OH in a light-shielded state.

The silica-coated fluorescent particles may be prepared at 10 to 90 °C, and preferably 75 to 85 °C.

Since the surfaces of the silica-coated fluorescent particles are coated with silica, the silica-coated fluorescent particles may be affected less by a variety of external factors. For example, when the fluorescent particles are directly bound to biological molecules, quenching may take place due to various external factors such as solvents, air, metals, salts and the like, and a decrease in degree of fluorescence may tend to occur with time. Since the silica-coated fluorescent particles are prepared by coating fluorescent particles with silica, the silica-coated fluorescent particles may serve to prevent any effects from the various external factors and continuously maintain an initial degree of fluorescence even with the passage of time. When the fluorescent particles are coated with silica in a similar manner, the fluorescent particles have merits in that they can be protected from photobleaching caused by laser beams used as a light source, and maintain a constant degree of fluorescence even when a change in pH occurs.

The binding of the at least one antibody of the antibiotic to the silica-coated fluorescent particles may include binding a carboxylic group to surfaces of the silica-coated fluorescent particles (S22), and forming an amide bond between the carboxylic group-bound silica-coated fluorescent particles and the antibody of the antibiotic (S24).

The binding of the carboxylic group to the surface of the silica-coated fluorescent particles (S22) may include mixing 3-APTES and TEOS with the silica-coated fluorescent particles while stirring, and dissolving succinic anhydride in dimethyl sulfoxide (DMSO), and mixing the resulting mixture with the silica-coated fluorescent particles while stirring.

The method according to the present invention may further include adding a cross-linking agent (S23) after binding the carboxylic group to the surfaces of the silica-coated fluorescent particles (S22). Here, the cross-linking agent may be selected from N-(3-dimethylaminopropyl)-N'-ethylcarbodiimide (EDC), N-hydroxysuccinimide (NHS), and a combination thereof.

The method according to the present invention may further include measuring an amount of the antibody of the antibiotic bound to the silica-coated fluorescent particles (S25) after binding the one or more antibodies of the antibiotics to the silica-coated fluorescent particles. Therefore, when an amount of the silica-coated fluorescent particles is measured using a fluorescence assay thereafter, an amount of the antibody of the antibiotic bound to the silica-coated fluorescent particles may be readily measured, which makes it possible to further reduce a detection limit of measuring a concentration of the antibiotic bound to the antibody of the antibiotic.

The measuring of the amount of the antibody of the antibiotic bound to the silica-coated fluorescent particles (S25) may include mixing a second fluorescent material, to which a secondary antibody specific to the antibody of the antibiotic is bound, with the silica-coated fluorescent particles, and measuring the first fluorescent material and the second fluorescent material using rays of light with different wavelengths. For example, when a degree of fluorescence of the first fluorescent material is measured using rays of light with first wavelengths, which are incident on the first fluorescent material, a degree of fluorescence of the second fluorescent material is measured using rays of light with second wavelengths, which are incident on the second fluorescent material, and the degrees of fluorescence of the first and second fluorescent materials are compared and analyzed, an amount of the antibiotic bound to the single silica-coated fluorescent particles may be measured.

The reaction of the magnetic particles with the silica-coated fluorescent particles (S3) may include performing an antigen/antibody reaction between the antibiotic bound to the magnetic particles and the antibody of the antibiotic bound to the silica-coated fluorescent particles. The antigen/antibody reaction is highly specific since this reaction is performed by selecting only an antigen against the antibody. Since the antibody of the antibiotic selects and reacts with only the antibiotic, the magnetic particles bound to the antibiotic may bind to the silica-coated fluorescent particles bound to the antibody of the antibiotic. Therefore, a degree of fluorescence of the silica-coated fluorescent particles may be directly associated with a concentration of the antibiotic.

After the reaction of the magnetic particles with the silica-coated fluorescent particles (S3), the method according to the present invention may further include collecting the magnetic particles, which have reacted with the silica-coated fluorescent particles, using a magnet (S4). When the magnetic particles are collected using the magnet, the antibiotic and the silica-coated fluorescent particles may be increased in concentration, which makes it favorable to measure a concentration of the antibiotic.

The collecting of the magnetic particles, which have reacted with the silica-coated fluorescent particles, using the magnet (S4) may be performed together with the collecting of the magnetic particles forming the amide bond with the antibiotics using the magnet (S 15), or one of the collecting operations may be performed.

The irradiating of the silica-coated fluorescent particles, which have reacted with the magnetic particles, with laser beams (S5) may further include measuring a degree of fluorescence of the silica-coated fluorescent particles using a fluorescence assay. The fluorescence assay may be, for example, an assay using a laser induced fluorescence microscope (LIFM).

The above-described method of measuring a concentration of an antibiotic can be useful in rapidly measuring a concentration of the antibiotic with inexpensive simple processes. Also, according to the above-described method of measuring a concentration of an antibiotic, a concentration of a target antibiotic to be measured may be accurately measured even when the target antibiotic is present at an extremely small amount. That is, a detection limit of measuring a concentration of an antibiotic using an enzyme-linked immunosorbent assay (ELISA) is approximately 1 ppb, but a detection limit of the method of measuring a concentration of an antibiotic according to one example embodiment of the present invention may be lowered to 0.045 ppb.

Hereinafter, the kit for measuring a concentration of an antibiotic according to one example embodiment of the present invention will be described. Since the kit for measuring a concentration of an antibiotic according to one example embodiment of the present invention is the kit used in the method of measuring a concentration of an antibiotic according to one example embodiment of the present invention, the differences in the two kits will be described for clarity.

The kit for measuring a concentration of an antibiotic includes a first measurement unit comprising magnetic particles to which a functional group capable of binding to an antibiotic is bound, and a second measurement unit comprising silica-coated fluorescent particles to which an antibody of the antibiotic is bound. In this case, the functional group capable of binding to the antibiotic may be an amine group.

The kit for measuring a concentration of an antibiotic is easily portable since the kit includes only the first measurement unit and the second measurement unit. As described above, the kit for measuring a concentration of an antibiotic may also be used to rapidly measure a very small concentration of the antibiotic with inexpensive simple processes.

Hereinafter, the present invention will be described in further detail by means of Preparative Examples, Experiment Examples, and Comparative Examples. However, it should be understood that the detailed described herein is given by way of illustration of the present invention only, and is not intended to limit the scope of the present invention.

### <Preparative Example 1> Manufacture of kit for measuring a concentration of enrofloxacin

### (1) Preparation of magnetic particles to which an amine group is bound

55 µl of 3-APTES and TEOS were added together to magnetic particles, and reacted for 24 hours while stirring to bind an amine group to surfaces of the magnetic particles.

### (2) Preparation of silica-coated FITC particles to which at least one antibody of enrofloxacin is bound

### ① Preparation of core particles including FITC (hereinafter referred to as "FITC particles")

10 mg of FITC was added to 10 ml of ethanol, and ultrasonicated for 5 minutes so as to dissolve the FITC in the ethanol completely. Thereafter, 50 µl of 3-APTES was added thereto, and agitated for 24 hours with a magnetic bar. The agitation was carried out in a state in which light was shielded with silver paper so that the FITC could avoid direct contact with light.

### ② Preparation of silica-coated FITC particles

5 mmol of docusate sodium salt and 860 µl of DIW were added to 48 ml of heptane, and agitated with a magnetic bar for 10 minutes to prepare a reverse microemulsion system. 300 µl of fluorescent particles including FITC was added to the reverse microemulsion system, and agitated with a magnetic bar for 30 minutes. Subsequently, 430 µl of TEOS and 270 µl of 25% NH₄OH serving as a catalyst were added, and reacted for 24 hours in a state in which light was shielded with silver paper.

### ③ Binding carboxylic group to surfaces of silica-coated FITC particles

55 µl of 3-APTES and TEOS were added together to silica-coated FITC particles, and reacted for 24 hours while stirring to activate an amine group on surfaces of the silica-coated FITC particles. Thereafter, to replace the amine group with a carboxylic group, a succinic anhydride was dissolved in DMSO, added to the silica-coated FITC particles, and reacted again for 2 hours while stirring.

### ④ Binding_ at least one antibody of enrofloxacin to silica-coated FITC particles to which a carboxylic group is bound

Cross-linking agents, EDC and NHS, were added together at a ratio of 2:1 to the carboxylic group-bound silica-coated FITC particles. Thereafter, 10 µl of an antibody of enrofloxacin was added, and reacted for 2 hours.

### <Preparative Example 2> Manufacture of kit for measuring a concentration of ciprofloxacin

A kit for measuring a concentration of ciprofloxacin was manufactured in substantially the same manner as in Preparative Example 1, except that an antibody of ciprofloxacin was used instead of the antibody of the enrofloxacin used in Preparative Example 1.

### <Preparative Example 3> Manufacture of kit for measuring an amount of an antibody bound to silica-coated fluorescent particles

### (1) Preparation of silica-coated Cy5 particles to which at least one antibody of

### enrofloxacin is bound

Silica-coated Cy5 particles to which at least one antibody of enrofloxacin was bound were prepared in substantially the same manner as in Preparative Example 1 (2) except that Cy5 was used instead of the FITC used in Preparative Example 1 (2).

### (2) Preparation of FITC to which a secondary antibody specific to enrofloxacin is bound

Cross-linking agents EDC and NHS were added together at a ratio of 2:1 to the FITC. Thereafter, 100 µl of a secondary antibody specific to an antibody of enrofloxacin was added, and reacted for 2 hours.

### <Preparative Example 4> Preparation of pretreatment sample

To remove proteins, acetonitrile was added to a sample of ground meat. The resulting mixture supplemented with a solvent was subjected to vortexing and ultrasonication while shaking for 10 minutes. A supernatant was collected, and hexane was added at the same volume ratio as acetonitrile so as to remove fats. Thereafter, the supernatant was subjected to vortexing and ultrasonication while shaking for 10 minutes. A fat layer was then removed. To remove the residual impurities, the impurities having a size of 0.2 µm or more were removed using a syringe filter.

### <Experiment Example 1> Stability test on silica-coated FITC particles

### (1) pH stability test

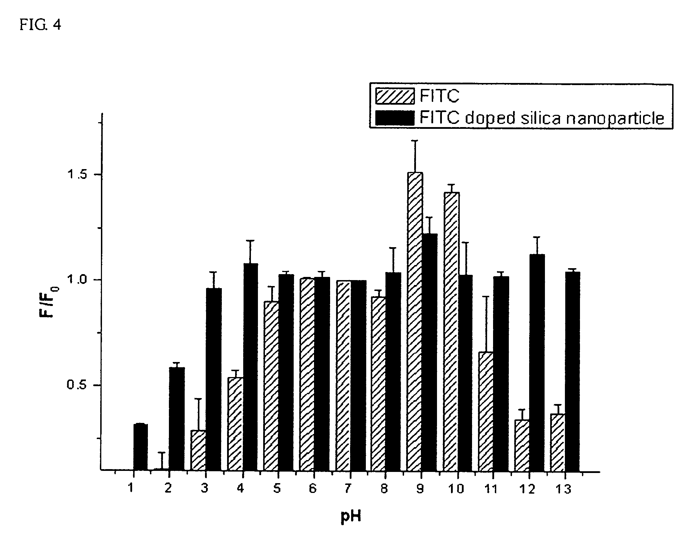

A vial was prepared, stored in 20 %HNO₃, and washed several times with DIW to remove metals present in the vial, or ions capable of having any effects. Solutions (pH 1 to 13) were put into a plurality of vials at doses of 1 ml. To prepare the solutions with pH 1 to 13, pH values of the solutions were adjusted with NaOH and HNO₃. 2 µl of the fluorescent particles, that is, FITC or silica-coated FITC particles, were added to the solutions, and reacted for an hour while rotating with a rotator. A degree of fluorescence was measured using an LIFM. The measurement results obtained in the pH stability test are shown in FIG. 4.

### (2) Photobleaching stability test

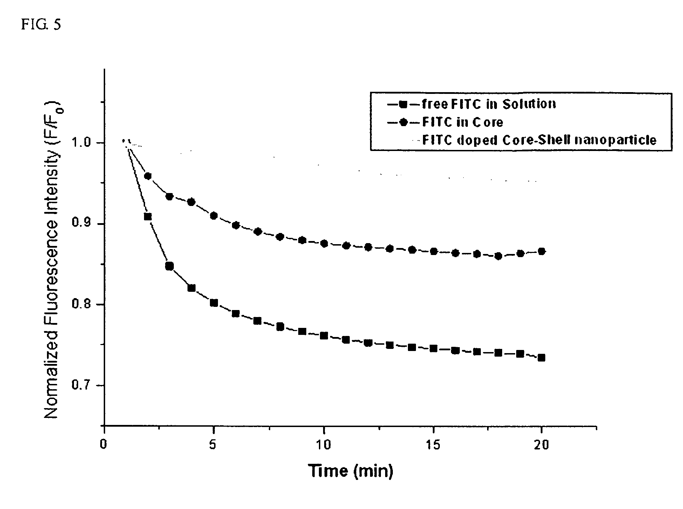

FITC was dissolved in ethanol, and diluted with DIW. Also, the FITC particles and the silica-coated FITC particles were diluted with DIW, and tested in the same manner as described, thereby preparing a sample. The sample was continuously irradiated with laser beams, and a fluorescence intensity was measured every one minute for a total of 20 minutes using a photomultiplier tube (PMT) and a photon counter. The measurement results obtained in the photobleaching stability test are shown in FIG 5.

### (3) Metal quenching stability test

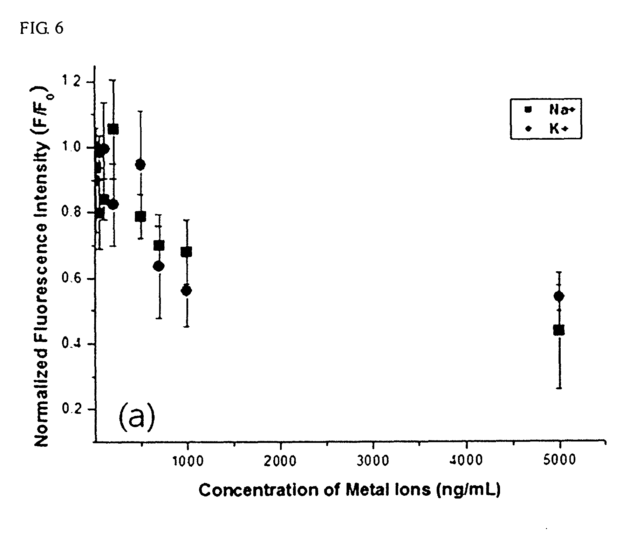

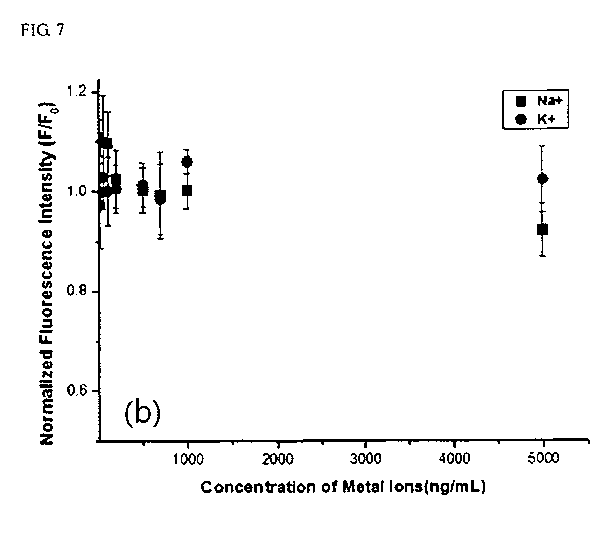

Concentrations of Na⁺ and K⁺ in the form of chloride ions were adjusted to 0, 10, 50, 100, 200, 500, 700, 1,000, and 5,000 µg/ml. The prepared metal solutions were put into vials at doses of 1 ml according to a concentration, and 2 µl of the fluorescent particles, that is, FITC or silica-coated FITC particles, were added to the vials, and thoroughly mixed. Degrees of fluorescence of the resulting mixtures were measured using an LIFM, a PMT, and a photon counter. Because FITC is not easily dissolved in DIW, FITC was dissolved in 1 mg/ml of ethanol, and 10 µl of the solution was diluted with 1 ml of DIW to be used. The measurement results obtained in the metal quenching stability test are shown in FIGS. 6 and 7. FIG 6 shows the measurement results of FITC in the metal quenching stability test, and FIG 7 shows the measurement results of the silica-coated FITC particles in the metal quenching stability test.

### <Experiment Example 2> Experiment for measuring an amount of antibody bound to silica-coated fluorescent particles

An amount of an antibody bound to silica-coated fluorescent particles was measured using the kit manufactured in Preparative Example 3. More particularly, a mixture including silica-coated Cy5 particles to which at least one antibody of enrofloxacin was bound and FITC to which a secondary antibody specific to an antibody of enrofloxacin was bound was prepared. The mixture was irradiated with 632.8 nm He/Ne red laser beams for measuring a degree of fluorescence of Cy5, and 473 nm diode-pumped solid-state (DPSS) blue laser beams using an LIFM. The degree of fluorescence of Cy5 representing an amount of the silica-coated Cy5 particles, and the degree of fluorescence of FITC representing an amount of the antibody of enrofloxacin were compared and analyzed. As a result, it was revealed that the antibody of enrofloxacin was present at a concentration of 5.28 nmol on surfaces of the silica-coated Cy5 particles.

### <Example 1> Experiment for measuring a concentration of enrofloxacin

When 10 ng/mL of enrofloxacin was measured with the kit for measuring a concentration of enrofloxacin prepared in Preparative Example 1,6.95 ng/mL of enrofloxacin was measured.

### <Example 2> Experiment for measuring a concentration of ciprofloxacin

When 10 ng/mL of ciprofloxacin was measured with the kit for measuring a concentration of ciprofloxacin prepared in Preparative Example 2, 7.34 ng/mL of ciprofloxacin was measured.

### <Comparative Example 1>

When 10 ng/mL of enrofloxacin was measured with a kit for measuring a concentration of enrofloxacin using an ELISA, 2.17 ng/mL of enrofloxacin was measured.

### <Comparative Example 2>

When 10 ng/mL of ciprofloxacin was measured with a kit for measuring a concentration of ciprofloxacin using an ELISA, 2.94 ng/mL of ciprofloxacin was measured.

According to the example embodiments of the present invention, the method and kit for measuring a concentration of an antibiotic have the following effects.

That is, a concentration of the antibiotic can be rapidly measured with inexpensive simple processes.

Also, an exact concentration of the antibiotic, which is present at an extremely small amount, can be measured.

The effects according to the present invention are not limited by the detailed description illustrated above, and a wider variety of effects are included in this specification.

While the example embodiments of the present invention and their advantages have been described in detail, it should be understood that various changes, substitutions and alterations may be made herein without departing from the scope of the invention.

## Claims

1. A method of measuring a concentration of an antibiotic, comprising:
preparing magnetic particles bound to an antibiotic;
preparing silica-coated fluorescent particles to which at least one antibody of the antibiotic is bound;
allowing the magnetic particles to react with the silica-coated fluorescent particles; and
irradiating the silica-coated fluorescent particles, which have reacted with the magnetic particles, with laser beams.

2. The method of claim 1, wherein the antibiotic is at least one antibiotic selected from the group consisting of enrofloxacin, ciprofloxacin, salinomycin, and sulfathiazole.

3. The method of claim 1, wherein the preparation of the magnetic particles bound to the antibiotic comprises:
binding an amine group to surfaces of the magnetic particles; and
forming an amide bond between the amine group-bound magnetic particles and the antibiotic.

4. The method of claim 3, further comprising:
adding a cross-linking agent after binding the amine group to the surfaces of the magnetic particles.

5. The method of claim 3, further comprising:
collecting the magnetic particles forming the amide bond with the antibiotic using a magnet after formation of the amide bond between the amine group-bound magnetic particles and the antibiotic.

6. The method of claim 1, wherein the preparation of the silica-coated fluorescent particles comprises:
forming silica-coated fluorescent particles by coating fluorescent particles including a first fluorescent material with silica; and
binding at least one antibody of the antibiotic to the silica-coated fluorescent particles.

7. The method of claim 6, wherein the first fluorescent material is at least one fluorescent material selected from the group consisting of fluorescein isothiocyanate, and cyanine.

8. The method of claim 6, wherein the binding of the antibody of the antibiotic to the silica-coated fluorescent particles comprises:
binding a carboxylic group to surfaces of the silica-coated fluorescent particles; and
forming an amide bond between the carboxylic group-bound silica-coated fluorescent particles and the antibody of the antibiotic.

9. The method of claim 8, further comprising:
adding a cross-linking agent after binding the carboxylic group to the surfaces of the silica-coated fluorescent particles.

10. The method of claim 6, further comprising:
measuring an amount of the antibody of the antibiotic bound to the silica-coated fluorescent particles after binding the one or more antibodies of the antibiotic to the silica-coated fluorescent particles.

11. The method of claim 10, wherein the measuring of the amount of the antibody of the antibiotic bound to the silica-coated fluorescent particles comprises:
mixing a second fluorescent material to which secondary antibodies specific to the antibody of the antibiotic are bound with the silica-coated fluorescent particles; and
measuring the first fluorescent material and the second fluorescent material using rays of light with different wavelengths.

12. The method of claim 1, further comprising:
collecting the magnetic particles, which have reacted with the silica-coated fluorescent particles, using a magnet after the reaction of the magnetic particles with the silica-coated fluorescent particles.

13. A kit for measuring a concentration of an antibiotic, comprising:
a first measurement unit comprising magnetic particles to which a functional group capable of binding to an antibiotic is bound; and
a second measurement unit comprising silica-coated fluorescent particles to which an antibody of the antibiotic is bound.

14. The kit of claim 13, wherein the functional group is an amine group.

15. The kit of claim 13, wherein the antibiotic is at least one antibiotic selected from the group consisting of enrofloxacin, ciprofloxacin, salinomycin, and sulfathiazole.

16. The kit of claim 13, wherein the silica-coated fluorescent particles are at least one fluorescent material selected from the group consisting of fluorescein isothiocyanate, and cyanine.

## Patentansprüche

1. Ein Verfahren zum Messen einer Konzentration eines Antibiotikums, das folgende Schritte aufweist:
Vorbereiten von magnetischen Partikeln, die an ein Antibiotikum gebunden sind;
Vorbereiten von Silika-beschichteten fluoreszierenden Partikeln, an die zumindest ein Antikörper des Antibiotikums gebunden ist;
Zulassen, dass die magnetischen Partikel mit den Silika-beschichteten fluoreszierenden Partikeln reagieren; und
Bestrahlen der Silika-beschichteten fluoreszierenden Partikel, die mit den magnetischen Partikeln reagiert haben, mit Laserstrahlen.

2. Das Verfahren gemäß Anspruch 1, bei dem das Antibiotikum zumindest ein Antibiotikum ist, das aus der Gruppe ausgewählt ist, die Enrofloxacin, Ciprofloxacin, Salinomycin und Sulfathiazol umfasst.

3. Das Verfahren gemäß Anspruch 1, bei dem die Vorbereitung der magnetischen Partikel, die an das Antibiotikum gebunden sind, folgende Schritte aufweist:
Binden einer Amingruppe an Oberflächen der magnetischen Partikel; und
Bilden einer Amidbindung zwischen den Amingruppe-gebundenen magnetischen Partikeln und dem Antibiotikum.

4. Das Verfahren gemäß Anspruch 3, das ferner folgenden Schritt aufweist:
Hinzufügen eines Vernetzungsmittels nach dem Binden der Amingruppe an die Oberflächen der magnetischen Partikel.

5. Das Verfahren gemäß Anspruch 3, das ferner folgenden Schritt aufweist:
Auffangen der magnetischen Partikel, die die Amidbindung mit dem Antibiotikum bilden, unter Verwendung eines Magnets nach Bildung der Amidbindung zwischen den Amingruppe-gebundenen magnetischen Partikeln und dem Antibiotikum.

6. Das Verfahren gemäß Anspruch 1, bei dem die Vorbereitung der Silika-beschichteten fluoreszierenden Partikel folgende Schritte aufweist:
Bilden von Silika-beschichteten fluoreszierenden Partikeln durch Beschichten von fluoreszierenden Partikeln, die ein erstes fluoreszierendes Material umfassen, mit Silika; und
Binden zumindest eines Antikörpers des Antibiotikums an die Silika-beschichteten fluoreszierenden Partikel.

7. Das Verfahren gemäß Anspruch 6, bei dem das erste fluoreszierende Material zumindest ein fluoreszierendes Material ist, das aus der Gruppe ausgewählt ist, die Fluoresceinisothiocyanat und Cyanin umfasst.

8. Das Verfahren gemäß Anspruch 6, bei dem das Binden des Antikörpers des Antibiotikums an die Silika-beschichteten fluoreszierenden Partikel folgende Schritte aufweist:
Binden einer Carboxylgruppe an Oberflächen der Silika-beschichteten fluoreszierenden Partikel; und
Bilden einer Amidbindung zwischen den Carboxylgruppe-gebundenen Silika-beschichteten fluoreszierenden Partikeln und dem Antikörper des Antibiotikums.

9. Das Verfahren gemäß Anspruch 8, das ferner folgenden Schritt aufweist:
Hinzufügen eines Vernetzungsmittels nach dem Binden der Carboxylgruppe an die Oberflächen der Silika-beschichteten fluoreszierenden Partikel.

10. Das Verfahren gemäß Anspruch 6, das ferner folgenden Schritt aufweist:
Messen einer Menge des Antikörpers des Antibiotikums, der an die Silika-beschichteten fluoreszierenden Partikel gebunden ist, nach dem Binden des einen oder der mehreren Antikörper des Antibiotikums an die Silika-beschichteten fluoreszierenden Partikel.

11. Das Verfahren gemäß Anspruch 10, bei dem das Messen der Menge des Antikörpers des Antibiotikums, der an die Silika-beschichteten fluoreszierenden Partikel gebunden ist, folgende Schritte aufweist:
Mischen eines zweiten fluoreszierenden Materials, an das für den Antikörper des Antibiotikums spezifische sekundäre Antikörper gebunden sind, mit den Silika-beschichteten fluoreszierenden Partikeln; und
Messen des ersten fluoreszierenden Materials und des zweiten fluoreszierenden Materials unter Verwendung von Lichtstrahlen mit verschiedenen Wellenlängen.

12. Das Verfahren gemäß Anspruch 1, das ferner folgenden Schritt aufweist:
Auffangen der magnetischen Partikel, die mit den Silika-beschichteten fluoreszierenden Partikeln reagiert haben, unter Verwendung eines Magnets nach der Reaktion der magnetischen Partikel mit den Silika-beschichteten fluoreszierenden Partikeln.

13. Eine Ausrüstung zum Messen einer Konzentration eines Antibiotikums, die folgende Merkmale aufweist:
eine erste Messeinheit, die magnetische Partikel aufweist, an die eine funktionelle Gruppe gebunden ist, die zur Bindung an ein Antibiotikum fähig ist; und
eine zweite Messeinheit, die Silika-beschichtete fluoreszierende Partikel aufweist, an die ein Antikörper des Antibiotikums gebunden ist.

14. Die Ausrüstung gemäß Anspruch 13, bei der die funktionelle Gruppe eine Amingruppe ist.

15. Die Ausrüstung gemäß Anspruch 13, bei der das Antibiotikum zumindest ein Antibiotikum ist, das aus der Gruppe ausgewählt ist, die Enrofloxacin, Ciprofloxacin, Salinomycin und Sulfathiazol umfasst.

16. Die Ausrüstung gemäß Anspruch 13, bei der die Silika-beschichteten fluoreszierenden Partikel zumindest ein fluoreszierendes Material sind, das aus der Gruppe ausgewählt ist, die Fluoresceinisothiocyanat und Cyanin umfasst.

## Revendications

1. Procédé de mesure de concentration d'un antibiotique, comprenant le fait de:
préparer des particules magnétiques liées à un antibiotique;
préparer des particules fluorescentes revêtues de silice auxquelles est lié au moins un anticorps de l'antibiotique;
permettre que les particules magnétiques réagissent avec les particules fluorescentes revêtues de silice; et
irradier par des faisceaux laser les particules fluorescentes revêtues de silice qui ont réagi avec les particules magnétiques.

2. Procédé selon la revendication 1, dans lequel l'antibiotique est au moins un antibiotique choisi parmi le groupe composé d'enrofloxacine, de ciprofloxacine, de salinomycine et de sulfathiazole.

3. Procédé selon la revendication 1, dans lequel la préparation des particules magnétiques liées aux antibiotiques comprend le fait de:
lier un groupe amine aux surfaces des particules magnétiques; et
former une liaison amide entre les particules magnétiques liées au groupe amine et l'antibiotique.

4. Procédé selon la revendication 3, comprenant par ailleurs le fait de:
ajouter un agent de réticulation après le fait de lier le groupe amine aux surfaces des particules magnétiques.

5. Procédé selon la revendication 3, comprenant par ailleurs le fait de:
collecter les particules magnétiques formant la liaison amide avec l'antibiotique à l'aide d'un aimant après le fait de former la liaison amide entre les particules magnétiques liées au groupe amine et l'antibiotique.

6. Procédé selon la revendication 1, dans lequel la préparation des particules fluorescentes revêtues de silice comprend le fait de:
former des particules fluorescentes revêtues de silice en revêtant les particules fluorescentes comportant un premier matériau fluorescent par de la silice; et
lier au moins un anticorps de l'antibiotique aux particules fluorescentes revêtues de silice.

7. Procédé selon la revendication 6, dans lequel le premier matériau fluorescent est au moins un matériau fluorescent choisi parmi le groupe composé d'isothiocyanate de fluorescéine, et de cyanine.

8. Procédé selon la revendication 6, dans lequel la liaison de l'anticorps de l'antibiotique aux particules fluorescentes revêtues de silice comprend le fait de:
lier un groupe carboxylique aux surfaces des particules fluorescentes revêtues de silice; et
former une liaison amide entre les particules fluorescentes revêtues de silice liées au groupe carboxylique et l'anticorps de l'antibiotique.

9. Procédé selon la revendication 8, comprenant par ailleurs le fait de:
ajouter un agent de réticulation après le fait de lier le groupe carboxylique aux surfaces des particules fluorescentes revêtues de silice.

10. Procédé selon la revendication 6, comprenant par ailleurs le fait de:
mesurer une quantité de l'anticorps de l'antibiotique lié aux particules fluorescentes revêtues de silice après le fait de lier les un ou plusieurs anticorps de l'antibiotique aux particules fluorescentes revêtues de silice.

11. Procédé selon la revendication 10, dans lequel la mesure de la quantité de l'anticorps de l'antibiotique lié aux particules fluorescentes revêtues de silice comprend le fait de:
mélanger un deuxième matériau fluorescent auquel sont liés des anticorps secondaires spécifiques à l'anticorps de l'antibiotique avec les particules fluorescentes revêtues de silice; et
mesurer le premier matériau fluorescent et le deuxième matériau fluorescent à l'aide de rayons de lumière de longueurs d'onde différentes.

12. Procédé selon la revendication 1, comprenant par ailleurs le fait de:
collecter les particules magnétiques qui ont réagi avec des particules fluorescentes revêtues de silice à l'aide d'un aimant après la réaction des particules magnétiques avec les fluorescentes revêtues de silice.

13. Kit de mesure d'une concentration d'un antibiotique, comprenant:
une première unité de mesure comprenant des particules magnétiques auxquelles est lié un groupe fonctionnel à même de se lier à un antibiotique; et
une deuxième unité de mesure comprenant des particules fluorescentes revêtues de silice auxquelles est lié un anticorps de l'antibiotique.

14. Kit selon la revendication 13, dans lequel le groupe fonctionnel est un groupe amine.

15. Kit selon la revendication 13, dans lequel l'antibiotique est au moins un antibiotique choisi parmi le groupe composé d'enrofloxacine, de ciprofloxacine, de salinomycine et de sulfathiazole.

16. Kit selon la revendication 13, dans lequel les particules fluorescentes revêtues de silice sont au moins un matériau fluorescent choisi parmi le groupe composé d'isothiocyanate de fluorescéine et de cyanine.
